Europäisches Patentamt

European Patent Office

Office européen des brevets

(19)

(11) **EP 0 993 832 A1**

(12) **EUROPEAN PATENT APPLICATION**

(43) Date of publication:
**19.04.2000 Bulletin 2000/16**

(51) Int Cl.7: **A61L 15/42**, A61L 15/58

(21) Application number: **98119581.1**

(22) Date of filing: **16.10.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant: **THE PROCTER & GAMBLE COMPANY**
**Cincinnati, Ohio 45202 (US)**

(72) Inventors:
 • **Carlucci, Giovanni**
  **66100 Chieti (IT)**

 • **Veglio, Paolo**
  **65129 Pescara (IT)**
 • **Bonelli, Guido**
  **65129 Pescara (IT)**
 • **Cimini, Carmine**
  **65126 Pescara (IT)**

(74) Representative:
**Kremer, Véronique Marie Joséphine et al**
**Procter & Gamble**
**European Service GmbH**
**65823 Schwalbach am Taunus (DE)**

(54) **Absorbent article comprising a microporous film with an adhesive layer**

(57) An absorbent article comprising a topsheet, a backsheet and an absorbent core therebetween is disclosed. The backsheet comprises a breathable microporous film with a layer of an adhesive composition, preferably a panty fastening adhesive composition, applied thereon. The adhesive composition is applied so as to show stable adhesive properties compensating the absorption of a migrating portion of the adhesive composition into the microporous film.

FIG. 1

EP 0 993 832 A1

**Description**

FIELD OF THE INVENTION

The present invention relates to a disposable absorbent article comprising a microporous film with an adhesive layer thereon having time stable adhesive properties. More specifically, the present invention relates to a disposable absorbent article with a backsheet comprising a breathable microporous film with a layer of panty fastening adhesive applied thereon having stable adhesive properties.

BACKGROUND OF THE INVENTION

Disposable absorbent articles such as sanitary napkins, pantiliners, and incontinence pads are devices that are typically worn in the crotch region of an undergarment and are intended to be discarded after a single use. These devices are designed to absorb and retain liquid and other discharges from the human body and to prevent body and clothing soiling. Sanitary napkins are a type of absorbent article worn by women in a pair of panties and are normally positioned between the wearer's legs, adjacent to the perineal area of the body.
Disposable absorbent articles comprising a microporous film are known. Microporous films having breathability are commonly used for various consumer products such as packaging films and disposable absorbent articles. There are prior art which are directed to improvement of such a microporous film, such as U. S. Patent 4,923,650 published on May 8, 1990, JP Patent publication 93/230252-A published on September 7, 1993, JP Patent publication 96/225680-A published on September 3, 1996, JP Patent publication 94/62794-B published on August 17, 1994, JP Patent publication 95/231913-A published on September 5, 1995, JP Patent publication 96/300436-A published on November 19, 1996, JP Patent publication 96/300498-A published on November 19, 1996, JP Patent publication 96/300499-A published on November 19, 1996, JP Patent publication 96/300500-A published on November 19, 1996, and JP Patent publication 87/167332-A published on July 23, 1987. The microporous film described in the prior art worked quite well as a backsheet of an absorbent article which requires breathability and liquid impermeability. There are also prior art which are directed to a process for making a microporous film and the microporous film made by the process, such as U.S. Patent 4,116,892 published on September 26, 1978, U. S. Patent 4,153,751 published on May 8, 1979, and U. S. Patent 4,289,831 published on September 15, 1981. These prior art disclose processes using a process of stretching a material to make a microporous film.
As mentioned above, microporous films are commonly used for a breathable backsheet of an absorbent article. Microporous films typically comprise a blend of a thermoplastic polymer and a filler dispersed therein, e.g. an inorganic filler such as calcium carbonate. The blend undergoes pore formation upon stretching in cross direction or in machine direction, or in both, as the inorganic filler separates from the polymer due to stress concentration. The formation of micropores permits the film to be breathable allowing the passage of vapour through the micropores while retarding the passage of liquid.
Disposable absorbent articles comprising a microporous film also usually comprise a layer of an adhesive composition applied on a surface of the microporous film, in order to join the microporous film to an adjacent layer. When the microporous film constitutes the preferably breathable backsheet of a sanitary napkin or pantiliner, typically a layer of a suitable adhesive is applied on the surface of the microporous film intended to face the wearer's undergarment in order to secure the sanitary napkin or pantiliner to the undergarment during the wearing time. Adhesive compositions that are usually applied to microporous films, for example hot melt pressure sensitive adhesive compositions applied as a panty fastening adhesive to the microporous film constituting the backsheet of a sanitary napkin or pantiliner, typically comprise at least one thermoplastic polymer, at least one tackifying resin, and at least one plasticiser, e.g. a mineral oil.
It has been discovered that preferred adhesive compositions comprise a migrating portion which is capable of being absorbed into the microporous film and tends to penetrate into the pores after application of the adhesive to the microporous film surface. This in turn modifies during time the composition of the adhesive and can impair its adhesive properties. Said migrating portion typically comprises lower molecular weight components of the adhesive, e.g. those having a molecular weight of less than 2000, more typically those components having a molecular weight of less than 1000, mainly comprising components of the at least one plasticiser, and also possibly of the at least one tackifying resin. It has been observed for example that a panty fastening adhesive applied to the microporous film constituting the backsheet of a sanitary napkin or pantiliner shows a reduced adhesiveness after the average storage time that is typical for these types of products, and therefore an effective securement of the absorbent article to the user's undergarment during wear by means of the panty fastening adhesive composition can be impaired, at least to a certain extent.
A possible solution to this problem is to apply the adhesive composition to the microporous film in such an amount that the adhesive properties of the composition are not reduced to an unacceptable level, according to the intended use of the adhesive, after a partial absorption of the migrating portion of the adhesive composition into the microporous film structure. It is of course desired to reduce to a minimum this extra amount of adhesive.
It is therefore an object of the present invention to provide an absorbent article comprising a microporous film with an adhesive composition applied thereon, wherein the adhesive composition comprises a migrating portion which is capable of being absorbed into the microporous film, in which the necessary amount of the adhesive composition can be predicted in order to have a desired residual adhesiveness, according to the intended use of the adhesive composition, after partial absorption of the migrating portion into the microporous film structure, also taking into account possible effects on the microporous film that could be induced during the manufacturing of the disposable absorbent article, for example due to a further stretching of the microporous film.

SUMMARY OF THE INVENTION

A disposable absorbent article comprising a microporous breathable film having a void volume percentage VV and a thickness TB, and an adhesive composition with time stable properties applied thereon, the adhesive composition comprising a migrating portion which is capable of being absorbed into the microporous breathable film, the migrating portion being present in the adhesive composition at a volume percentage VP, the microporous breathable film being capable of absorbing the migrating portion in an amount proportional to the void volume VV according to a fraction factor FF of absorbency of the film for the migrating portion,

the adhesive composition being applied onto the microporous breathable film in a layer having a thickness TP defined by the equation:

$$TP \geq TB \cdot \frac{VV \cdot FF}{0.47 \cdot VP}$$

the microporous breathable film comprising a thermoplastic resin and a filler dispersed therein, e.g. an inorganic filler, and being stretched at least in machine direction in order to be provided with breathability.

BRIEF DESCRIPTION OF THE DRAWINGS

FIG. 1 is a bottom plan view of a preferred sanitary napkin embodiment of the present invention.
FIG. 2 is a lateral cross-sectional view taken along line 2-2 of Figure 1.
FIG. 3 is a fragmentary enlarged cross-sectional view of a part of the absorbent core and a part of the backsheet of the sanitary napkin.

DETAILED DESCRIPTION OF THE INVENTION

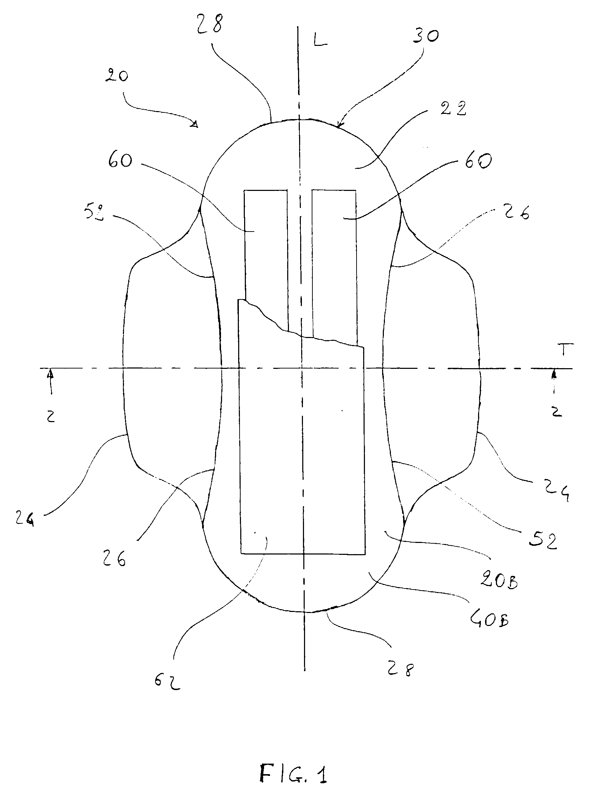

A preferred embodiment of the present invention is shown in FIG. 1 which illustrates a sanitary napkin 20. As shown in FIG. 1, the sanitary napkin 20 basically comprises an absorbent means (or "main body portion") 22, and two flaps 24. The sanitary napkin 20 has two surfaces, a body-contacting surface or "body surface" 20A and a garment surface 20B. The sanitary napkin 20 is shown in FIG. 1 as viewed from its garment surface 20B. The body surface 20A is intended to be worn adjacent to the wearer's body. The garment surface 20B is intended to be placed adjacent to the wearer's undergarments when the sanitary napkin 20 is worn. The sanitary napkin 20 has two centerlines, a principal longitudinal centerline L and a principal transverse centerline T.

FIG. 1 shows that the main body portion 22 of the sanitary napkin 20 comprises the portion of the sanitary napkin without the flaps 24. The main body portion 22 has two spaced apart longitudinal edges 26, two spaced apart transverse or end edges (or "ends") 28, which together form the periphery 30 of the main body portion. The main body portion 22 of the sanitary napkin 20 can be of any thickness, including relatively thick, intermediate thickness, relatively thin, or even very thin (or "ultra thin"). An "ultra-thin" sanitary napkin 20 as described in U.S. Patents 4,950,264 and 5,009,653 issued to Osborn preferably has a caliper of less than about 3 millimeters. The embodiment of the sanitary napkin 20 shown in the drawings is intended to be an example of a sanitary napkin of an intermediate thickness. The main body portion 22 of the sanitary napkin 20 may also be relatively flexible, so that it is comfortable for the wearer. It should be understood that the sanitary napkin shown is merely one embodiment, and that the present invention is not limited to absorbent articles of the type or having the specific configurations shown in the drawings.

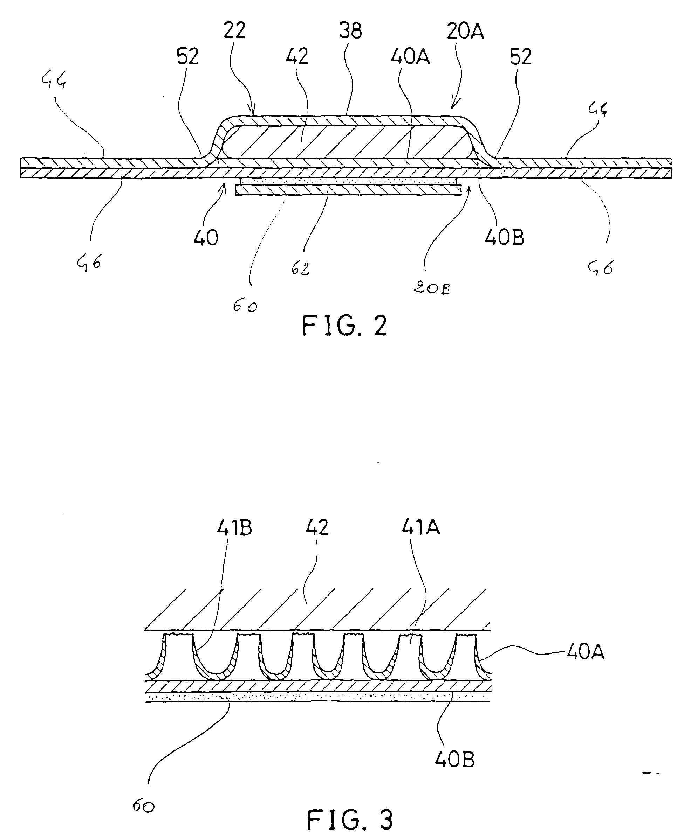

FIG. 2 shows the individual components of the main body portion 22 of the sanitary napkin 20 of the present invention. The main body portion 22 of the sanitary napkin 20 preferably comprises at least three primary components. These include a liquid pervious topsheet 38 typically provided by a liquid permeable substrate of fibrous such as nonwoven or film like structure such as apertured formed films, a liquid impervious backsheet 40 preferably provided by a liquid impermeable, but breathable substrate, and an absorbent core 42 positioned between the topsheet 38 and the backsheet 40. The backsheet 40 comprises two layers; a first layer comprising a gas permeable apertured formed film layer 40A and a second layer comprising a breathable microporous film layer 40B.
The topsheet, the backsheet, and the absorbent core may be assembled in a variety of configurations known in the art (including layered or "sandwich" configurations and wrapped or "tube" configurations). FIGS. 1 and 2 show a preferred embodiment of the sanitary napkin 20 assembled in a sandwich construction in which the topsheet 38 and the breathable microporous film 40B have length and width dimensions generally larger than those of the absorbent core 42. The topsheet 38 and the breathable microporous film 40B extend beyond the edges of the absorbent core 42 to form portions of the periphery 30. The apertured formed film 40A of the backsheet has the approximately same shape as the absorbent core 42 to cover at least the region where the absorbent core 42 lies as shown in FIG. 2. Alternatively, it may have a little bigger shape than the absorbent core 42, or may have the same shape as the main body portion 22 of the sanitary napkin 20. In any case, preferably, the apertured formed film 40A does not extend into the flaps 24 as shown in FIG. 2. Alternatively, the apertured formed film 40A may extend into the flaps 24 so that the apertured formed film constitutes a part of the flaps 24.

The topsheet 38 is preferably joined to the body-facing side of the absorbent core 42 and the backsheet 40, specifically, the apertured formed film 40A of the backsheet 40 is preferably joined to the garment-facing side of the absorbent core 42. The topsheet 38 and the apertured formed film 40A can be joined to the absorbent core 42 in any suitable manner known in the art for this purpose, such as by an open pattern of adhesives. The portions of the topsheet 38 and the breathable microporous film 40B that extend beyond the edges of the absorbent core are preferably also joined to each other. The topsheet 38 and the breathable microporous film 40B can be joined in any suitable manner known in the art for this purpose. Preferably, in the embodiment shown, these portions of the topsheet 38 and the breathable microporous film 40B are joined using adhesives over substantially the entire portions that extend beyond the edges of the absorbent core 42, and a crimp seal at the end edges 28 of the main body portion where the topsheet 38 and backsheet 40 are densified by the application of pressure or heat and pressure.

The sanitary napkin 20 shown in FIGS. 1 and 2 also comprises a pair of flaps 24 that are joined to the main body portion 22 along a juncture, such as lines of juncture 52. The flaps 24 extend laterally outward beyond the longitudinal side edges 26 of the main body portion 22 from their proximal edges to their distal edges (or "free ends"). The flaps 24 comprise a flap topsheet 44 and a flap backsheet 46. In the embodiment shown in FIGS. 1 and 2, the flaps 24 are integral with the main body portion 22, that is, the flap topsheet 44 and the flap backsheet 46 comprise integral extensions of the topsheet 38 and the breathable microporous film 40B, respectively. In the preferred embodiment, the apertured formed film 40A does not extend into the flaps 24.

The extensions of the topsheet 38 and the breathable microporous film 40B of the flaps 24 (i.e., the flap topsheet 44 and the flap backsheet 46) may be joined by any suitable method, such as adhesive attachment, ultrasonic attachment, heat attachment or the like. In the preferred embodiment, the extensions of the topsheet 38 and the breathable microporous film 40B are joined by applying adhesive to substantially all the region of the flaps 24.

The function of side flaps, whether integral or joined to the article after being formed separately, can be further improved by rendering them extensible in one or both directions parallel to the longitudinal centreline L or transverse centreline T. The extensibility can be provided across all or only part of the side flaps and can be achieved by joining an elastic material to the side flap (for elastication and extensibility) or pleating or ring-rolling those parts which are to be rendered extensible.

## Topsheet

The topsheet 38 is preferably compliant, soft feeling, and non-irritating to the wearer's skin. Further, the topsheet 38 is fluid pervious, permitting fluid to readily penetrate through its thickness. A suitable topsheet 38 may be manufactured from a wide range of materials such as woven and nonwoven materials; polymeric materials such as apertured formed thermoplastic films, apertured plastic films, and hydroformed thermoplastic films; porous foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers); or from a combination of natural and synthetic fibers. A preferred topsheet comprises an apertured formed film. In the preferred embodiment of the present invention, apertured formed films are preferably used for the topsheet because they are pervious to body exudates and yet non-absorbent and have a reduced tendency to allow fluids to pass back through and rewet the wearer's skin. Thus, the surface of the formed film which is in contact with the body remains dry, thereby reducing body soiling and creating a more comfortable feel for the wearer. Suitable apertured formed films are described in U.S. Patent No. 3,929,135 issued to Thompson, on December 30, 1975; U.S. Patent No. 4,324,246 issued to Mullane et al., on April 13, 1982; U.S. Patent No. 4,342,314 issued to Radel, et al., on August 3, 1982; U. S. Patent No. 4,463,045, issued to Ahr, et al., on July 31, 1984 and U.S. Pat. No. 5,006,394 issued to Baird, on April 9, 1991. A preferred topsheet for the present invention comprises the formed film described in one or more of the above patents and marketed on sanitary napkins by The Procter & Gamble Company of Cincinnati, Ohio as "DRIWEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

Absorbent core

The absorbent core 42 may be any absorbent means which is generally compressible, conformable, resilient, non-irritating to the wearer's skin and capable of absorbing and containing body exudates. The absorbent core 42 may be manufactured from a wide variety of fluid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibers (such as those described in U.S. Patent No. 5,217,445 issued to Young, et al., on June 8, 1993), capillary channel fibers (that is, fibers having intra-fiber capillary channels such as those described in U.S. Patent No. 5,200,248 issued to Thompson, et al., on April 6, 1993), absorbent foams (such as those described in U.S. Patent No. 5,260,345 issued to DesMarais, et al., on November 9, 1993 and U.S. Patent No. 5,268,244 issued to DesMarais, et al., on December 7, 1993), thermally bonded airlay materials (such as those material described in U.S. Patent No. 5,607,414 issued to Richards, et al., on March 4, 1997), hydrogel-forming polymer gelling agents (such as those material described in U.S. Patent No. 4,673,402 issued to Weisman, et al., on June 16, 1987 and U.S. Patent No. 4,935,022 issued to Lash et al., on June 19, 1990), absorbent sponges, synthetic staple fibers, polymeric fibers, peat moss, or any equivalent materials or combinations of materials. Further, the absorbent core 42 may comprise a first portion and a second portion, the first portion comprising the following components: (a) an optional primary fluid distribution layer preferably together with a secondary optional fluid distribution layer; (b) a fluid storage layer; and the second portion comprising (c) an optional fibrous layer underlying the storage layer; and (d) other optional components. Such a structure is disclosed in PCT publication WO 97/24096 published on July 10, 1997 and WO 97/24095 published on July 10, 1997.

Another component which can be included in the absorbent structure according to the invention and preferably is provided close to or as part of the primary or secondary fluid distribution layer are odour control agents. Typically active carbon coated with or in addition to other odour control agents, in particular suitable zeolite or clay materials, are optionally incorporated in the absorbent structure. These components can be incorporated in any desired form but often are included as discrete particles.

Backsheet

The backsheet 40 is preferably impervious to liquid and pervious to vapour. The primary role of the backsheet 40 is to prevent the exudates absorbed and contained in the absorbent core 42 from wetting articles that contact the absorbent product such as underpants, pants, pajamas and undergarments. In addition however, the backsheet 40 also permits the transfer of both vapour and air through it and thus allows the circulation of air into and out of the backsheet 40.

In the embodiment shown in Fig. 2, the backsheet 40 comprises two layers; a first layer comprising a gas permeable apertured formed film layer 40A and a second layer comprising a breathable microporous film layer 40B. The first layer 40A is typically located adjacent to the absorbent core 42 and subsequent layers of the backsheet are typically located further away from the absorbent core 42. The backsheet 40 may comprise additional layers. All of the layers of the backsheet 40 can be substantially in intimate and direct contact with one another.

As shown in FIG. 3 which shows an enlarged cross sectional view of the backsheet 40 with a part of the absorbent core 42, the first layer of the apertured formed film 40A comprises a layer having discrete apertures 41A which extend beyond the horizontal plane of the garment facing surface of the layer towards the absorbent core 42 thereby forming protuberances 41B. Each protuberance 41B has an orifice located at its terminating end. Preferably the protuberances 41B have a funnel or conical shape, similar to those described in US 3,929,135. The apertures located within the plane of the layer and the orifices located at the terminating end of protuberances themselves maybe circular or non circular. In any case the cross sectional dimension or area of the orifice at the termination of the protuberance is smaller than the cross sectional dimension or area of the aperture located within the plane of the layer. The first layer 40A of the backsheet 40 may be made of any material known in the art, but is preferably manufactured from commonly available polymeric materials. The first layer 40A may also comprise any type of formed films which may be used for a topsheet as described above.

The second layer 40B of the backsheet 40 may comprise a breathable microporous film composed of a thermoplastic resin and a filler, e.g. an inorganic filler, dispersed in the thermoplastic resin. Suitable thermoplastic polymers include polyolefins such as polyethylenes, including linear low density polyethylene (LLDPE), low density polyethylene (LDPE), ultra low density polyethylene (ULDPE), high density polyethylene (HDPE), or polypropylene and blends thereof with the above and other materials. Examples of other suitable thermoplastic polymers which may also be used include, but are not limited to, polyester, polyurethanes, compostable or biodegradable polymers, thermoplastic elastomers, and metallocene catalyst-based polymers (e.g., INSITE® available from Dow Chemical Company and Exxact® available from Exxon). The filler can be preferably an inorganic material and can comprise talc, silica, calcium carbonate, clay, titanium dioxide, barium sulfate, with the preferred inorganic filler being calcium carbonate. The inorganic filler may be coated with fatty acid esters, fatty acids or their metal salts to improve the dispersion of the filler particles into the thermoplastic polymer and to obtain higher loadings in the polymer. The preferably inorganic filler and the thermoplastic polymer are blended together to form a homogeneous mixture in a suitable mixing extruder, or in a separate preliminary compounding step. The mixture is then cast or blown into a film. The obtained film is stretched at least in one direction to impart breathability on the substantially entire area of the film. The step of stretching a film to impart breathability may be done at a different place prior to manufacturing process of absorbent articles. Alternatively, the step of stretching may be done at the same place, i.e., same manufacturing process, prior to assembling a breathable microporous film with other elements of absorbent articles. In any case, the film is imparted breathability on the substantially entire area of the film before the resulting breathable microporous film is assembled with other elements of absorbent articles. According to the present invention, the microporous film is stretched in at least the machine direction in order to be provided with breathability, preferably in both the machine direction and the cross direction.

Panty fastening system

In the sanitary napkin 20 that represents a preferred embodiment of the present invention the microporous film has an adhesive composition applied thereon that acts as a panty fastening adhesive, but according to the present invention the adhesive composition applied to the microporous film can also serve different scopes, e.g. as a construction adhesive used to keep different components forming the disposable absorbent article together.

According to the present invention, the sanitary napkin 20 is fastened to the undergarment by means of a layer of a panty fastening adhesive composition 60 applied on the garment surface 20B of the microporous film layer 40B of the backsheet 40. The layer of panty fastening adhesive 60 provides a means for securing the absorbent article to the panty and preferably a means for securing the absorbent article when soiled to the fold and wrap package for convenient disposal. Typically, at least a portion of the garment facing surface 20B of the microporous film layer 40B, preferably a portion corresponding to the front portion of the absorbent core 42 only, is coated with adhesive to form the panty fastening adhesive. A panty fastening adhesive can also be applied onto the garment facing surface of the flaps of a sanitary napkin. Adhesives or glues used in the art for such purposes, for example NS 34-2823 manufactured by the National Starch and Chemical Company of Bridgewater, could be used for the panty fastening adhesive herein.

The panty fastening adhesive 60 is typically applied to the microporous film layer 40B of the backsheet 40 by slot coating or spraying in various distribution patterns, such as e.g. continuous or discontinuous strips, intermittent dots, random patterns spirals. In the embodiment of the present invention illustrated in FIG. 1 the panty fastening adhesive 60 is applied as a continuous layer by means of a slot coater, in two longitudinal stripes symmetrically parallel to the longitudinal centreline L.

The panty fastening adhesive 60 is typically covered with a removable release paper or film 62 in order to prevent the adhesive from drying out or adhering to another surface other than the panty prior to use. Any commercially available release paper or film may be used. Suitable examples include BL 30MG-A SILOX El/O and BL 30 MG-A SILOX 4 P/O available from Akrosil Corporation.

According to the present invention, the microporous breathable film layer 40B comprised in the backsheet 40 of the sanitary napkin 20 has a thickness TB and a void volume percentage VV, that, for this preferred use in a breathable backsheet for disposable absorbent articles, can typically range from 30% to 60%. The void volume VV is usually provided by the film manufacturer, or can be determined with methods known in the art. The adhesive composition applied onto the garment facing surface 20B of the microporous film layer 40B usually comprises at least one thermoplastic polymer, at least one tackifying resin, and at least one plasticiser, e.g. a mineral oil. The adhesive composition comprises a migrating portion which is capable of being absorbed into the microporous film, i.e., it can be absorbed into the pores of the microporous film layer owing to a mechanism including capillarity and also affinity with the particles of the preferably inorganic filler included in the microporous film structure. The migrating portion typically comprises lower molecular weight components of the adhesive, e.g. those having a molecular weight of less than 2000, more typically those components having a molecular weight of less than 1000, mainly comprising components of the at least one plasticiser, and also possibly of the at least one tackifying resin. The migrating portion is present in the adhesive composition in a volume percentage VP that can be directly determined from the composition of the adhesive composition, or alternatively with methods known in the art. The volume of the migrating portion of the adhesive composition absorbed into the void volume of the microporous film at equilibrium, as measured according to the procedure described hereafter, is proportional to the void volume VV of the microporous film according to a fraction factor FF.

The adhesive composition 60 has to be applied to the microporous film 40B in a layer having a thickness TP defined by the equation:

$$TP \geq TB \cdot \frac{VV \cdot FF}{0.47 \cdot VP}$$

wherein the expression at the denominator represents the highest fraction of the whole migrating portion volume percentage VP of the adhesive composition that has been found can be actually absorbed into the microporous film without impairing the final pressure sensitive adhesive properties of the adhesive composition, for most uses of said adhesive compositions, e.g. preferably as panty fastening adhesive in a sanitary napkin or pantiliner. This highest fraction has been empirically found to correspond to a 47% of the volume percentage VP of the migrating portion for most uses of the adhesive composition applied onto a microporous breathable film.

The identification of this highest allowable fraction of the volume percentage VP of the migrating portion that can be absorbed into the microporous film without impairing the final adhesive characteristics of the adhesive composition, which in turn depend on the intended use of the adhesive, has been achieved experimentally by measuring the residual adhesiveness, in terms of peel force according to the test method described hereinafter, of samples having different amounts of adhesive composition applied onto a microporous breathable film, said peel force being measured after the samples have been subjected to the Accelerated Aging Procedure, also described hereinafter, that simulates the effects on the adhesive composition applied onto a microporous film of a long storage period of a disposable absorbent article, and basically corresponds to an absorption of the migrating portion of the adhesive composition into the microporous film at equilibrium conditions. Typically the peel force measured after the Accelerated Aging Procedure represents the substantially constant residual value after equilibrium conditions in the absorption of the migrating portion of the adhesive composition into the microporous film have been attained, and can be also defined as the equilibrium peel force.

The amount of adhesive composition applied to the microporous breathable film is such that, after equilibrium absorption of the migrating portion of the adhesive composition in the pores of the microporous film has occurred, which involves a modification of the composition of the adhesive layer, such adhesive composition still retains an adhesive capacity which is sufficient for the intended use of the adhesive itself. Such adhesive capacity can be easily determined by the man skilled in the art according to the scope intended for the adhesive composition applied onto the microporous film, e.g. as a structural adhesive used for keeping together the structure of the disposable absorbent article, or, as it is preferred, as a panty fastening adhesive.

The void volume of a microporous film incorporated into a disposable absorbent article, e.g. preferably as a breathable backsheet into a sanitary napkin or pantiliner, can be influenced and possibly modified with respect to the void volume originally imparted to the microporous film by stretching during the manufacturing of the microporous film itself, by the further stress to which a microporous breathable film is likely to be subjected during the manufacturing process of a disposable absorbent article that incorporates such a film. The void volume of the microporous film incorporated into the final product is relevant to the evaluation of the amount of migrating portion that can be absorbed at equilibrium, i.e., after the Accelerated Aging Procedure, into such a specific microporous breathable film comprised in the product, and therefore of the fraction factor FF that must be related to the void volume VV of the microporous film in order to determine the amount of adhesive composition to be applied onto a microporous breathable film according to the above described equation.

However, the microporous breathable film can have its void volume originally imparted by the manufacturer modified in an unpredictable and not consistent way by the stress, specifically by a certain amount of machine direction stretching, to which it is subjected during the process for manufacturing a disposable absorbent article incorporating the microporous film. This in turn makes a determination of the fraction factor FF uncertain, since it would relate to an actual value of the void volume of the microporous film in the absorbent article which can be, and most likely is, different from the void volume of the microporous film as such after its production.

It has been discovered that if the microporous breathable film is produced by stretching it in the machine direction in order to be provided with breathability, or, in other words, if the microporous breathable film is stretched in a direction corresponding to the machine direction in the subsequent manufacturing of the absorbent article comprising said film, this uncertainty is eliminated, and the stress of the microporous film during the manufacture of the product, typically resulting from a further slight stretch in machine direction, does not substantially influence the original void volume of the microporous film, or, in any case, its effect on said void volume is limited and consistent for a given microporous film and for a given process for manufacturing the absorbent article incorporating the film. Alternatively, even if less preferably, the microporous breathable film used in the manufacture of an absorbent article according to the present invention can also be stretched in both the machine direction and the cross direction in order to be provided with breathability.

In a preferred embodiment of the present invention a dual layer backsheet 40 having the same arrangement as that described with reference to FIGS. 1 to 3, and comprising an apertured formed film layer 40A and a microporous breathable film 40B providing a fluid impervious, water vapour pervious breathable backsheet 40, has been incorporated into a sanitary napkin similar to the Always Ultra marketed by The Procter & Gamble Company, wherein the dual layer breathable backsheet replaces the traditional polyethylene backsheet.

A suitable microporous film, e.g. that available from Mitsui under the tradename Espoir PG-01, can have a void volume of 45% (VV=45) and a thickness of 40 μm (TB=0.004 cm), and has been stretched in machine direction to be provided with breathability.

A layer of a hot melt pressure sensitive adhesive is applied as the panty fastening adhesive to the garment face of the microporous film. The adhesive is applied by means of slot coater in two longitudinal stripes symmetrically parallel to the longitudinal centreline L, and centered with respect to the transverse centreline T, each stripe comprising a continuous homogeneous layer having a constant thickness TP. Each stripe is 2.3 cm wide and 17.1 cm long, and the two stripes are separated by a distance of 1.1 cm. The adhesive used has a migrating portion of about 75% in volume (VP=75), corresponding to the total percentage in volume of the components having a molecular weight of less than 2000, and can be for example that available from National Starch under the code NS-34-2823. The panty fastening adhesive is covered by a release paper.

The amount of migrating portion absorbed at equilibrium into the microporous film, expressed in terms of the fraction factor FF of the void volume VV of the microporous film, has been determined as follows. Samples are prepared from a continuous strip 12 cm wide of the Espoir PG-01 microporous film mentioned above, onto which a layer of the selected adhesive composition is applied in two stripes having the same arrangement and dimensions as described above. The adhesive is applied in each of the two stripes as a continuous layer having a basis weight of 25 $g/m^2$, by means of a suitable coater, e.g. an Acumeter Model LH-1 extruder. The samples are cut from the continuous strip in such a length that each sample comprises the two stripes of adhesive in their full length.

Two series of samples are prepared: Sample 1 (reference sample) is a sample as described above taken soon after its preparation, i.e. within 24 hours from application of the adhesive composition onto the microporous film. Sample 2 is a sample of the same type as Sample 1, which has been subjected to the Accelerated Aging Procedure as described hereinafter.

The two samples are weighed on a suitable precision scale; the weight of the microporous film plus the adhesive composition applied thereon is then recorded and of course is the same for the two samples.

The adhesive composition is then removed from the surface of the microporous film of the two samples by a thorough cleaning with a suitable solvent, e.g. with a cotton wad wetted with dichloromethane, until no residual stickiness can be detected on the microporous film surface. The lack of residual stickiness on the microporous film can be easily ascertained for example by pressing a flat polished steel plate onto the cleaned surface of the microporous film, with the film lying on a flat surface: the condition is satisfied if, when lifting vertically the steel plate, the microporous film does not stick at all to the steel plate and remains on the surface. Care should be taken to avoid that the cleaning action with the solvent eliminates also part of the migrating portion of the adhesive composition of Sample 2 that has been absorbed into the microporous film. To reduce this possible effect to a negligible extent a cotton wad is passed along the entire length of an adhesive stripe only once with a continuous wiping movement and is then discarded and replaced with a new one, until no residual stickiness is left on the surface of the microporous film. The cotton wads could be preferably large enough to cover the width of a single adhesive stripe, and should also be wetted with the suitable solvent, rather than soaked.

The two samples are weighed again, and the difference of the two weight values (in grams) corresponds to the amount of the migrating portion of the adhesive composition actually absorbed at equilibrium into the pores of the microporous film of Sample 2 during the Accelerated Aging Procedure. Each result is averaged on ten test samples. In the preferred example of the present invention described so far, the amount of migrating portion absorbed at equilibrium into the microporous film backsheet corresponds to 8.5 $g/m^2$, with respect to the area actually covered by the adhesive composition, independently of the amount of adhesive composition applied to the microporous film.

The fraction factor FF may then be easily calculated for Sample 2 as a function of the actual void volume of the microporous film (in $cm^3$) and of the amount of migrating portion absorbed in this void volume, through the density of the migrating portion that can be easily determined with methods known in the art, which in the present case is about 0.92 $g/cm^3$. The value of FF for Sample 2 is 0.51.

When the values determined so far are substituted in the equation described above the minimum thickness TB of the adhesive layer to be applied as panty fastening adhesive onto the microporous backsheet of the sanitary napkin according to the present invention is TB ≥ 0.026 mm. With such a minimum amount, the partial absorption of the migrating portion of the adhesive composition into the microporous film during a standard average storage time of the absorbent article, simulated by the Accelerated Aging Procedure, does not modifies the properties of the adhesive composition to such an extent that the adhesiveness of the panty fastening adhesive goes below the commonly acceptable level for this type of usage, represented by a peel force on cotton of at least 80 g.

Accelerated Aging Procedure.

According to the Accelerated Aging Procedure, a complete disposable absorbent article according to the present invention is transferred right after production, i.e. not later than 24 hours after application of the adhesive onto the microporous film, into an environment at 40°C and 75% relative humidity, and is kept there for 4 weeks.

Peel Force Test.

The peel force test analyses the force required to delaminate a connection between materials when one material is peeled from the other material at a 180 degree angle. The test is based on the standard test method ASTM D 1876 - 72, but with a head speed of the tension testing machine of 100 mm/min, the samples being prepared as described hereinafter.

The test is performed on samples that have been previously subjected to the Accelerated Aging Procedure, and then are tested after a conditioning of at least 2 hours at the test conditions.

In the determination of the residual adhesiveness of the adhesive composition applied as a panty fastening adhesive onto a microporous film constituting the backsheet of a sanitary napkin, each sample is constituted by an entire product as described above.

The sample is positioned onto a flat horizontal surface with the garment facing surface upwards, then the release paper is removed and a substrate layer constituted by a woven cotton fabric is applied onto the adhesive layer. The substrate should extend the full surface of the adhesive layer and preferably exceed this surface sufficiently to provide edges where it can be manipulated and fixed to the clamp of the tension testing machine. The woven cotton fabric can be obtained under the designation "white, 100% cotton weave, style # 429-W, available from Loeffler Sitter Technik GmbH, Nettersheim, Germany. A pressure is applied to the entire surface of the adhesive layer with a 3.5 kg weight for 30 seconds.

The sample is then connected to the tension testing machine for the measurement of the peel force necessary to delaminate the connection between the substrate and the adhesive layer when the substrate is peeled from the sample at a 180 degree angle, and with a head speed of the tension testing machine of 100 mm/min. This corresponds to the sample being connected to the fixed clamp, and the cotton fabric connected to the moving clamp at its free edge. The average force (in grams) measured is the actual force necessary to delaminate the cotton substrate from the panty fastening adhesive of the sanitary napkin, and is not converted into a force for a given width of the adhesive connection.

The test procedure described so far, derived from the ASTM D1876 - 72 standard test method, has proven to be effective in the measurement of the strength of the adhesive connection performed by the panty fastening adhesive in a sanitary napkin towards a cotton substrate, which represents a preferred embodiment of the present invention, but of course the same standard method can be suitably modified by the skilled man in order to be adapted to alternative embodiments falling within the scope of the present invention, e.g. when the adhesive layer applied onto the microporous film constitutes a construction adhesive in the structure of a disposable absorbent article.

In the sanitary napkin of the present invention described so far and illustrated in FIGS. 1 to 3, the backsheet 40 comprises a microporous film layer 40B and an apertured formed film 40A interposed between the microporous film layer 40B and the absorbent core 42, and located adjacent to the absorbent core 42. This constitutes a particularly preferred embodiment of the present invention. It has been in fact also discovered that, if the surface of the microporous layer which is opposite to the surface on which the adhesive composition is applied, corresponding in the present case to the body facing surface of the microporous film, i.e. the surface facing towards the user's body, is in direct contact with a layer having an affinity for the migrating portion contained in the adhesive composition, for example a fibrous layer or an absorbent element such as the core of a sanitary napkin, the migrating portion of the adhesive composition after reaching an equilibrium condition with the void spaces of the microporous film can also be absorbed by the adjacent layer. This involves that an overall equilibrium condition is very likely not attained until almost the whole amount of the migrating portion of the adhesive composition is actually absorbed by the combined action of the microporous film and of the adjacent absorbing fibrous layer.

The presence between the microporous layer and an adjacent absorbent layer of an intermediate layer which is not capable of absorbing the migrating portion of the adhesive composition avoids this effect. Preferably, in order to keep the breathability of the structure, a breathable non absorbent layer is interposed to separate the microporous film from an adjacent absorbent layer, such as for example the apertured formed film 40A of the dual layer composite breathable backsheet 40 of the sanitary napkin 20 of the present invention.

**Claims**

1. A disposable absorbent article comprising a microporous breathable film having a void volume percentage VV and a thickness TB, and an adhesive composition with time stable properties applied on a first surface thereof, said adhesive composition comprising a migrating portion which is capable of being absorbed into said microporous breathable film, said migrating portion being present in said adhesive composition at a volume percentage VP, said microporous breathable film being capable of absorbing said migrating portion in an amount proportional to said void volume VV according to a fraction factor FF of absorbency of said film for said migrating portion,

said adhesive composition being applied onto said microporous breathable film in a layer having a thickness TP defined by the equation:

$$TP \geq TB \cdot \frac{VV \cdot FF}{0.47 \cdot VP}$$

said microporous breathable film comprising a thermoplastic resin and a filler, preferably an inorganic filler, dispersed therein, and being stretched at least in machine direction in order to be provided with breathability.

2.   A disposable absorbent article according to claim 1, wherein said migrating portion comprises components of said adhesive composition having a molecular weight of less than 2000.

3.   A disposable absorbent article according to claim 2, wherein said migrating portion comprises components of said adhesive composition having a molecular weight of less than 1000.

4.   A disposable absorbent article according to any preceding claim, wherein said disposable absorbent article is a sanitary napkin comprising a topsheet, a backsheet and an absorbent core comprised therebetween, said backsheet being breathable and comprising said microporous breathable film, said adhesive composition constituting a panty fastening adhesive for said sanitary napkin.

5.   A disposable absorbent article according to claim 4, wherein said adhesive composition has a peel force of at least 80 g on a cotton substrate as measured after the Accelerated Aging Procedure and according to the Peel Force Test, both the Accelerated Aging Procedure and the Peel Force Test being described herein.

6.   A disposable absorbent article according to any preceding claim, wherein said disposable absorbent article comprises an absorbent layer adjacent to said microporous breathable film on a second surface thereof opposed to said first surface, said disposable absorbent article further comprising a breathable layer which is not capable of absorbing said migrating portion, said breathable layer separating said microporous breathable film from said adjacent absorbent layer.

FIG. 1

FIG. 2

FIG. 3

| | European Patent Office | EUROPEAN SEARCH REPORT | Application Number |
|---|---|---|---|
| | | | EP 98 11 9581 |

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | WO 95 27516 A (KAO CORP ;SUZUKI MIKIO (JP); TAKEUCHI KEN (JP); WADA MASASHI (JP)) 19 October 1995<br>* page 2, line 8 - page 4, line 14 *<br>* page 4, line 22 - page 5, line 12 *<br>* page 5, line 21 - page 6, line 10 *<br>* page 15, line 10 - page 17, last line *<br>* page 20, line 21 - page 21, line 8 *<br>* page 22, line 16 - last line *<br>* page 28, line 1 - page 29, line 8 *<br>* figure 5 *<br>* claims 1-7 * | 1-6 | A61L15/42<br>A61L15/58 |
| Y | EP 0 779 325 A (MITSUI TOATSU CHEMICALS) 18 June 1997<br>* page 3, line 26 - line 33 *<br>* page 3, line 44 - line 51 *<br>* page 4, line 8 - line 27 *<br>* page 5, line 39 - page 6, line 1 *<br>* page 6, line 13 - line 19; tables 1,2 *<br>see claims | 1-6 | |
| A | PATENT ABSTRACTS OF JAPAN<br>vol. 016, no. 304 (C-0959), 6 July 1992<br>& JP 04 084960 A (KAO CORP),<br>18 March 1992<br>* abstract * | 1-4 | **TECHNICAL FIELDS SEARCHED (Int.Cl.6)**<br><br>A61L |
| A | DATABASE WPI<br>Section Ch, Week 9217<br>Derwent Publications Ltd., London, GB;<br>Class A18, AN 92-136848<br>XP002096989<br>& JP 04 077591 A (KANEBO NSC KK)<br>, 11 March 1992<br>* abstract * | 1-3 | |
| | | -/-- | |

The present search report has been drawn up for all claims

| Place of search | Date of completion of the search | Examiner |
|---|---|---|
| THE HAGUE | 18 March 1999 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**European Patent Office**

Application Number

EP 98 11 9581

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | DATABASE WPI<br>Section Ch, Week 9627<br>Derwent Publications Ltd., London, GB;<br>Class A17, AN 96-263937<br>XP002096990<br>& JP 08 109360 A (UBE IND LTD)<br>, 30 April 1996<br>* abstract * | 1-3 | |

TECHNICAL FIELDS SEARCHED (Int.Cl.6)

The present search report has been drawn up for all claims

| Place of search | Date of completion of the search | Examiner |
|---|---|---|
| THE HAGUE | 18 March 1999 | Thornton, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 0 993 832 A1

## ANNEX TO THE EUROPEAN SEARCH REPORT
## ON EUROPEAN PATENT APPLICATION NO.

EP 98 11 9581

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-1999

| Patent document cited in search report | | Publication date | Patent family member(s) | | Publication date |
|---|---|---|---|---|---|
| WO 9527516 | A | 19-10-1995 | AU | 684158 B | 04-12-1997 |
| | | | AU | 2084695 A | 30-10-1995 |
| | | | CN | 1126949 A | 17-07-1996 |
| | | | EP | 0702571 A | 27-03-1996 |
| | | | JP | 8033677 A | 06-02-1996 |
| | | | US | 5738669 A | 14-04-1998 |
| EP 0779325 | A | 18-06-1997 | JP | 9221560 A | 26-08-1997 |

EPO FORM P0459

For more details about this annex : see Official Journal of the European Patent Office, No. 12/82

15